# DEMANDE DE BREVET EUROPEEN

(11) **EP 1 243 250 A1**
(43) Date de publication de la demande: **25.09.2002**
(21) Numéro de dépôt: 02290535.0
(22) Date de dépôt: 05.03.2002
(51) Int. Cl.: A61K 7/48

(54) **Utilisation de fibres comme agent anti-irritant dans une composition cosmétique ou dermatologique**

(30) Priorité: 23.03.2001 FR 0103956
(71) Demandeur: L'OREAL, 75008 Paris (FR)
(72) Inventeur: Creton, Isabelle, 75005 Paris (FR)
(74) Mandataire: Rasson, Catherine

(57) **Abrégé**

La présente invention concerne l'utilisation de fibres, comme agent anti-irritant dans une composition pour application topique, et pour diminuer ou supprimer l'effet irritant d'un ou plusieurs composés à effet secondaire irritant, dans une composition cosmétique ou dermatologique.

Elle se rapporte aussi à une composition pour application topique, notamment cosmétique ou dermatologique, comprenant des fibres et un ou plusieurs composés à effet secondaire irritant, et un procédé de traitement cosmétique mettant en oeuvre la composition cosmétique selon l'invention.

## Description

La présente invention concerne l'utilisation de fibres comme agent anti-irritant dans une composition pour application topique, et pour diminuer l'effet irritant d'un ou plusieurs composés à effet secondaire irritant, dans une composition cosmétique, ou pour la préparation d'une composition dermatologique contenant ce ou ces composés.

Elle concerne également une composition pour application topique comprenant un ou plusieurs composés à effet secondaire irritant et des fibres, ainsi qu'une composition pour application topique contenant des fibres et un agent apaisant. Elle concerne aussi un procédé de traitement cosmétique mettant en oeuvre lesdites compositions.

Dans le cadre de la présente invention, l'effet secondaire irritant est un effet irritant cutané (ou une irritation cutanée), et c'est une réponse de la peau se traduisant le plus souvent par des rougeurs, douleurs ou picotements, cette réponse étant engendrée par des composés chimiques d'origine naturelle ou synthétique, appliqués de manière topique sur la peau. Cette irritation s'accompagne d'une altération de la fonction et/ou de la structure épithéliale, directement liées à l'effet du composé à caractère irritant.

Ainsi, les perturbations induites par un composé à caractère irritant sont suivies par une réponse plus ou moins intense de la peau, visant à restaurer l'équilibre homéostatique rompu ou à réparer les dommages provoqués. Lorsque le composé à caractère irritant a atteint la peau, il peut réagir avec certaines substances préexistantes dans les cellules et les tissus et/ou libérer des substances intracellulaires. Ces substances libérées peuvent à leur tour devenir actives sur d'autres cibles dans l'épithélium ou le derme. Ainsi, s'amorce la cascade des réactions qui, par le recrutement de cellules sanguines et les substances qu'elles libèrent, donnent naissance au processus irritant qui se caractérise principalement par une irritation de la peau. Ce processus se traduit notamment à des degrés divers, dépendant principalement de la qualité et/ou de la quantité du composé appliqué et/ou de l'utilisateur de ce composé, par des sensations dysesthétiques (échauffement, sensations de brûlures, démangeaisons ou prurit, sensations de picotements, de tiraillements, etc...), par des rougeurs et/ou par un oedème.

Des composés à effet secondaire irritant, c'est-à-dire pouvant provoquer une irritation cutanée, spécialement pour les personnes (utilisateurs) à peau sensible, sont présents dans des compositions cosmétiques ou dermatologiques, bien entendu pour d'autres effets.

Ainsi, on utilise des compositions cosmétiques contenant des actifs kératolytiques, pour lutter contre le vieillissement, et notamment des actifs exfoliants ou des actifs favorisant le renouvellement cellulaire, tels que les α-hydroxy-acides (notamment acides lactique, glycolique, citrique), les β-hydroxy-acides (notamment acides salicylique, n-octanoyl-5-salicylique) et les rétinoïdes (notamment acide rétinoïque tout trans ou 13-cis, rétinol). Malheureusement, si ces actifs sont utilisés en des quantités trop importantes, ils présentent l'inconvénient de provoquer des picotements, des démangeaisons, des tiraillements après leur application, pouvant conduire à un important inconfort. L'utilisation de ces composés notamment pour les utilisateurs à peau sensible doit donc être limitée.

En outre, même certains composés qui sont considérés comme inertes dans une composition cosmétique ou dermatologique, tels que par exemple les conservateurs, les tensioactifs, les parfums, les solvants ou les propulseurs, peuvent présenter un caractère irritant lorsqu'ils sont appliqués sur les matières kératiniques et notamment la peau y compris le cuir chevelu, ce caractère irritant dépendant du composé utilisé et de la sensibilité de la peau de l'utilisateur. Ainsi, il est fréquent d'utiliser des agents tensioactifs, émulsionnants ou détergents, dans les compositions cosmétiques ou dermatologiques, par exemple comme émulsionnants dans des compositions se présentant sous forme d'émulsions, eau-dans-huile (E/H) ou huile-dans-eau (H/E) ou triple, ou par exemple comme détergents dans des compositions de nettoyage de la peau humaine. De même, il est fréquent d'incorporer des conservateurs pour assurer une bonne conservation des compositions, ou des parfums pour donner une odeur agréable aux produits.

Les composés ayant un caractère irritant sont généralement utilisés en des doses faibles. L'utilisation à faible quantité de ces composés peut alors s'avérer peu avantageuse par rapport à l'utilisation d'autres composés moins actifs, mais moins ou pas irritants et donc utilisés en plus grande quantité, ou par rapport à la finalité du composé, telle que par exemple, la stabilité de la composition quand il s'agit d'émulsionnants ou la bonne conservation de la composition quand il s'agit de conservateurs.

Il subsiste donc le besoin, dans les domaines cosmétique et dermatologique, de trouver un moyen permettant d'utiliser ces composés, sans que ces derniers présentent un caractère irritant reprochable par l'utilisateur, et donc d'obtenir une composition cosmétique ou dermatologique qui, bien que contenant des composés à effet secondaire irritant, soit confortable et non irritante.

Or, la Demanderesse a découvert que les fibres permettent de limiter, voire de supprimer, le caractère irritant de ces composés.

Certes, le document EP-A-1,057,477 décrit une composition contenant des fibres de cellulose, la présence de ces fibres permettant d'éviter l'utilisation d'émulsionnants. Du fait de l'absence d'émulsionnants, la composition obtenue est moins irritante, mais ce document ne divulgue pas que les fibres permettent de diminuer l'irritation d'un agent irritant présent dans la composition.

Ainsi, la présente invention a pour objet l'utilisation cosmétique de fibres comme agent anti-irritant dans une composition cosmétique.

L'invention se rapporte encore à l'utilisation de fibres comme agent anti-irritant pour la préparation d'une composition pour application topique destinée à traiter les matières kératiniques.

L'invention se rapporte encore à l'utilisation d'une quantité efficace de fibres pour diminuer l'effet irritant d'une composition cosmétique contenant un ou plusieurs composés à effet secondaire irritant.

La présente invention a aussi pour objet l'utilisation d'une quantité efficace de fibres pour la préparation d'une composition dermatologique contenant un ou plusieurs composés à effet secondaire irritant, les fibres étant destinées à diminuer cet effet irritant.

La présente invention présente notamment l'avantage de supprimer l'irritation qu'auraient pu provoquer les composés à effet secondaire irritant, et aussi de permettre d'augmenter la quantité de composés à caractère irritant dans des compositions cosmétiques ou dermatologiques par rapport à la quantité normalement utilisée, en vue d'une efficacité de ces derniers améliorée. Ainsi, on peut utiliser les tensioactifs, les conservateurs ou les actifs kératolytiques tels que les hydroxyacides ou les rétinoïdes sans aucune gène pour l'utilisateur.

Ainsi, la présente invention se rapporte aussi à une composition pour application topique, notamment cosmétique ou dermatologique, caractérisée en ce qu'elle comprend des fibres et un ou plusieurs composés à effet secondaire irritant choisi parmi les α-hydroxy-acides, les β-hydroxy-acides, les α-céto-acides, les β-céto-acides, les anthralines, les anthranoïdes, les peroxydes, le minoxidil et ses dérivés, les sels de lithium, les agents antitranspirants, les actifs dépilatoires ou de permanentes, les dépigmentants, la capsaïcine, les actifs antipoux, les tensioactifs anioniques, et leurs mélanges.

On entend ici par « application topique » une application externe sur les matières kératiniques, et par « matières kératiniques », on entend notamment la peau, le cuir chevelu, les cils, les sourcils, les ongles et les muqueuses.

Selon un mode particulier de réalisation de l'invention, la composition contient au moins deux composés à effet secondaire irritant, par exemple deux, trois, quatre composés à effet secondaire irritant, ou plus, par exemple deux actifs et un tensioactif, ou encore deux actifs, un tensioactif, et un solvant.

La composition selon l'invention étant destinée à une application topique comprend un milieu physiologiquement acceptable, c'est-à-dire compatible avec toutes les matières kératiniques telles que la peau y compris le cuir chevelu, les ongles, les muqueuses, les yeux et les cheveux ou toute autre zone cutanée du corps. Elle peut constituer notamment une composition cosmétique ou dermatologique.

La présente invention a également pour objet un procédé cosmétique pour diminuer ou supprimer l'effet irritant d'au moins un composé à effet secondaire irritant présent dans une composition cosmétique, caractérisé en ce que l'on utilise une quantité efficace de fibres.

La quantité efficace de fibres selon l'invention est une quantité suffisante de fibres pour diminuer voire supprimer un effet irritant cutané. Ainsi, cette quantité est variable en fonction des fibres utilisées, de la quantité et de la nature du composé à caractère irritant utilisé et/ou de la sensibilité de l'utilisateur à ce composé. Cependant, à titre d'illustration, une composition selon l'invention comprend généralement des fibres à une concentration allant de 0,01 à 50 % en poids, de préférence de 1 à 20 % en poids et mieux de 5 à 10 % en poids par rapport au poids total de la composition.

Outre un bon effet anti-irritant, les compositions contenant les fibres ont de bonnes propriétés cosmétiques : confort à l'application, texture facile à étaler et douce.

Les fibres utilisables dans la composition de l'invention peuvent être des fibres hydrophiles ou hydrophobes, d'origine synthétique ou naturelle, minérale ou organique.

Ces fibres peuvent être courtes ou longues, unitaires ou organisées par exemple tressées. Leur forme ou morphologie peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres peuvent avoir une longueur (L) allant de 1 µm (0,001 mm) à 10 mm, de préférence de 0,1 µm à 5 mm et mieux de 0,1 mm à 1,5 mm. Leur section peut être comprise dans un cercle de diamètre (D) allant de 1 nm (0,001 µm) à 100 µm, de préférence allant de 1 nm (0,001 µm) à 50 µm et mieux de 5 µm à 40 µm.

De préférence, les fibres utilisées selon la présente invention ont un facteur de forme, c'est-à-dire un rapport L/D (longueur/diamètre) allant de 3,5 à 2500, mieux de 5 à 500 et encore mieux de 5 à 150.

Le titre des fibres est souvent donné en denier ou décitex. Le denier est le poids en gramme pour 9 km de fil. De préférence, les fibres utilisées selon l'invention ont un titre allant de 0,15 à 30 deniers, et mieux de 0,18 à 18 deniers.

Le facteur de forme, le titre et la morphologie des fibres sont les trois facteurs importants pour définir une fibre.

Les fibres peuvent être celles utilisées dans la fabrication des textiles et notamment des fibres de soie, de coton, de laine, de lin, de cellulose extraites notamment du bois, des légumes ou des algues, de polyamide (Nylon®), de cellulose modifiée (rayonne, viscose, acétate notamment d'acétate de rayonne), de poly-p-phénylène téréphtalamide notamment de Kevlar®, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-dessus, comme des fibres de polyamide/polyester.

Comme fibres de polyuréthane, on peut citer par exemple les fibres en polymère poly(uréthane-urée), appartenant à la classe des élastanes, et notamment celles commercialisées sous la dénomination LYCRA® par la société DuPont.

On peut aussi utiliser les fibres synthétiques résorbables utilisées en chirurgie, comme les fibres préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson) et les fils d'acier inoxydable (Acier de la société Johnson & Johnson).

On peut aussi utiliser des mélanges des fibres citées ci-dessus.

Par ailleurs, les fibres peuvent être traitées ou non en surface, enrobées ou non. Il peut s'agir notamment de fibres enrobées et/ou fonctionnalisées, « fonctionnalisées » signifiant que les fibres sont traitées en surface afin d'en modifier les propriétés.

Comme fibres enrobées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou traitement de surface induisant des effets de couleurs/hologrammes (fibre Lurex de la société Sildorex, par exemple).

Les fibres peuvent être également fonctionnalisées, c'est-à-dire être modifiées de manière à avoir une fonction spécifique. Cette fonctionnalisation des fibres peut être réalisée aussi bien sur les fibres que dans les fibres, et ce par toute'méthode permettant d'accrocher un composé aux fibres ou de le piéger dans les cavités formées par la géométrie des fibres. Comme méthodes, on peut citer par exemple l'enduction des fibres par un actif ; la fixation de particules renfermant un actif, telles que nanocapsules ou nanosphères, sur les fibres ; l'adsorption dans les fibres ; la fixation par réaction chimique. On peut ainsi utiliser des fibres ayant des fonctionnalités particulières, par exemple des fibres anti-UV par modification avec filtres solaires chimiques ou physiques ; des fibres bactéricides ou antiseptiques par modification avec des conservateurs ou d'anti-bactériens ; des fibres colorées par modification avec des molécules colorantes ; des fibres kératolytiques ou desquamantes par modification avec des agents kératolytiques ou desquamants ; des fibres hydratantes par modification avec des agents hydratants ou des polymères rétenteurs d'eau ; les fibres parfumées par modification avec un parfum ; des fibres analgésiques ou apaisantes par modification avec un anti-inflammatoire ou un agent apaisant ; des fibres anti-transpirantes par modification avec un anti-transpirant.

Selon leurs propriétés, les fibres utilisées selon la présente invention peuvent être introduites dans un milieu aqueux, un milieu huileux ou dans une poudre.

Les fibres utilisables selon l'invention sont de préférence choisies parmi les fibres de polyamide, les fibres de poly-p-phénylène téréphtalamide, les fibres de coton et leurs mélanges. Leur longueur peut aller de 0,1 à 10 mm, de préférence de 0,1 à 1 mm, leur diamètre moyen peut aller de 5 à 50 µm et le facteur de forme va de préférence de 5 à 150.

En particulier, on peut utiliser les fibres de polyamide commercialisées par les Etablissements P. Bonte sous le nom Polyamide 0,9 dtex 0,3 mm, ayant un diamètre moyen de 15 à 20 µm, un titre d'environ 0,9 dtex (0,81 denier) et une longueur allant de 0,3 mm à 1,5 mm. On peut aussi utiliser les fibres de poly-p-phénylène téréphtalamide de diamètre moyen de 12 µm et de longueur d'environ 1,5 mm comme celles vendues sous le nom de Kevlar Floc par la société Du Pont Fibres. Ces fibres de polyamide sont de préférence introduites dans un milieu huileux ou par voie sèche dans une poudre.

On peut aussi utiliser des fibres de coton ayant un diamètre moyen de 20 µm, une longueur de 0,3 mm, et un facteur de forme de 15, telles que celles commercialisées par la société Filature de Lomme, par la société Textiles des Dunes ou par la société Velifil.

Comme indiqué ci-dessus, les fibres peuvent être présentes dans la composition selon l'invention en une quantité allant par exemple de 0,01 à 50 % en poids, de préférence de 1 à 20 % en poids et mieux de 5 à 10 % en poids de matière active par rapport au poids total de la composition.

Les composés à effet secondaire irritant et donc susceptibles de provoquer une irritation cutanée, spécialement pour les personnes à peau sensible, peuvent être notamment des actifs, des conservateurs, des tensioactifs, des parfums, des solvants, des propulseurs, et leurs mélanges.

Comme actifs susceptibles d'avoir un effet secondaire irritant, on peut citer par exemple :
- les agents kératolytiques tels que les α-hydroxy-acides comme les acides glycolique, lactique, malique, citrique, tartrique, mandélique, et leurs dérivés ; les β-hydroxy-acides comme l'acide salicylique et ses dérivés ; les α-céto-acides comme l'acide ascorbique ou vitamine C et ses dérivés ; les β-céto-acides; les rétinoïdes comme le rétinol et ses esters, le rétinal, l'acide rétinoïque et ses dérivés, ceux décrits dans les documents FR-A-2 570 377, EP-A-199 636, EP-A-325 540, EP-A-402 072) ;
- les anthralines (dioxyanthranol) ;
- les anthranoïdes (par exemple ceux décrits dans le document EP-A- 319028) ;
- les peroxydes (notamment le peroxyde de benzoyle) ;
- le minoxidil et ses dérivés ;
- les sels de lithium ;
- les teintures ou colorants capillaires comme la para-phénylène diamine (p-PDA) et certains de ses dérivés tels que la N-phenyl p-PDA et le toluène 2,5-diamine sulfate ; la méta-phénylène diamine (m-PDA) et certains de ses dérivés tels que la toluène 3,4-diamine ; l'ortho-phénylène diamine (o-PDA) ;
- les solutions alcooliques parfumantes (parfums, eaux de toilette, après rasage, déodorants) ;
- les agents antitranspirants (certains sels d'aluminium) ;
- les actifs dépilatoires ou de permanentes (thiols, ammoniaque) ;
- les dépigmentants (hydroquinone) ;
- la capsaïcine ;
- les actifs antipoux (pyréthrine) ;
- les agents détergents (ioniques et non ioniques) ;
- et leurs mélanges.

Parmi les dérivés de l'acide salicylique, on peut citer plus particulièrement l'acide n-octanoyl-5-salicylique, l'acide n-dodécanoyl-5-salicylique et leurs esters.

Comme conservateurs, on peut citer le phénoxyéthanol, et les bactéricides tels que l'octoxyglycérine ou 1-2(éthylhexyl)-glycérol éther (SENSIVA SC 50 commercialisé par la société Seppic), et le caprylyl-glycol (ou 1,2-octanediol) commercialisé par la société Straetmans.

Comme tensioactifs, on peut citer plus particulièrement les tensioactifs anioniques tels que les alkyl sulfates et alkyl éther sulfates comme le lauryl sulfate et le lauryl éther sulfate, et leurs sels notamment de sodium.

La composition peut contenir un ou plusieurs composés à caractère irritant, qui peuvent avoir la même activité ou une activité différente, par exemple un tensioactif, un actif et un conservateur, ou un conservateur et un tensioactif.

De préférence, la quantité du ou des composés à effet secondaire irritant dans la composition de l'invention peut être suffisante pour provoquer une irritation cutanée, les fibres permettant d'atténuer voire supprimer cette irritation. Cette quantité dépend du ou des composés utilisés et de la finalité de la composition le ou les contenant. Elle peut aller par exemple de 0,0001 à 50 % en poids, de préférence de 0,01 à 30 % en poids et mieux de 0,1 à 15 % en poids par rapport au poids total de la composition.

Le milieu physiologiquement acceptable des compositions à application topique, selon l'invention peut être plus particulièrement constitué d'eau et éventuellement d'un solvant organique physiologiquement acceptable choisi par exemple parmi les alcools inférieurs comportant de 1 à 8 atomes de carbone et de préférence 1 à 6 atomes de carbone, comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol.

Ce milieu peut être aussi un milieu anhydre, notamment un milieu huileux contenant des huiles et/ou matières grasses autres que les huiles.

Quand le milieu physiologiquement acceptable est aqueux, il a de préférence un pH compatible avec la peau, allant de préférence de 3 à 8 et mieux de 4,5 à 7.

Quand la composition comporte un milieu aqueux ou hydroalcoolique, il est possible d'ajouter une phase grasse (ou huileuse) dans ce milieu, afin que les compositions de l'invention soient plus douces et plus nourrissantes.

La phase huileuse contient habituellement au moins une huile. Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoylés et notamment éthoxylés tels que l'oleth-12 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals; le perfluoro-1,2-diméthylcyclobutane; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluorométhoxybutane vendu sous la dénomination "MSX 4518®" par la Société 3M et le nonafluoroéthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les compositions selon l'invention peuvent se présenter sous toutes les formes galéniques classiquement utilisées pour une application topique et notamment sous forme de solutions aqueuses, hydroalcooliques ou huileuses, d'émulsions huile-dans-eau (H/E) ou eau-dans-huile (E/H) ou multiple, de gels aqueux ou huileux, de produits anhydres liquides, pâteux ou solides, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique. Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90^{R} par la société Goldschmidt.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose.

Selon un mode particulier de réalisation de l'invention, on associe aux fibres utilisées selon l'invention, un ou plusieurs actifs apaisants choisis parmi les autres agents anti-irritants et/ou les agents anti-inflammatoires, ces agents apaisants venant renforcer l'effet anti-irritant des fibres.

Ainsi, l'invention a aussi pour objet une composition pour application topique, contenant dans un milieu physiologiquement acceptable, des fibres et au moins un agent apaisant choisi parmi l'allantoïne ; l'acide bêta-glycyrrhétinique et les extraits et complexes en contenant ; les planctons ; l'escine et les extraits végétaux en contenant ; les dérivés de xanthine ; les eaux et extraits de fleurs et de plantes ; l'acide asiatique et les extraits végétaux en contenant ; le bisabololol ; les extraits de fruits ; les algues ; les pyrrolidone carboxylates ; les huiles d'origine végétale ; les huiles essentielles ; l'acide acexamique et l'acide transexamique ; l'acide ursolique et les extraits en contenant ; les polysaccharides contenant du fucose ; les électrolytes ; les galactolipides ; les acides aminés, leurs dérivés et leurs sels ; les antagonistes de TNF-alpha ; les antagonistes de substance P ; et leurs mélanges.

Ainsi qu'indiqué ci-dessus, les agents apaisants utilisés selon la présente invention peuvent être choisis parmi:
- l'allantoïne ;
- l'acide bêta-glycyrrhétinique, les extraits en contenant comme par exemple l'extrait de Glycyrrhiza Glabra (réglisse) et les complexes en contenant comme le complexe allantoïne/acide glycyrrhétinique ;
- les planctons, lyophilisés ou non, leurs extraits et leurs complexes ;
- l'escine et les extraits végétaux en contenant comme l'extrait de marron d'inde ;
- les dérivés de xanthine comme le chlorhydrate de di-éthylaminoéthyl théophylline ;
- les eaux et extraits (par exemple aqueux, hydroalcooliques ou hydroglycoliques) de fleurs et de plantes, comme l'eau de bleuet, l'eau de camomille, l'eau de menthe, l'eau de tilleul, l'eau de rose, les extraits de pivoine, les extraits d'aubépine, les extraits de millefeuille, les extraits de mauve, les extraits de souci, les extraits de melilot, les extraits de sauge, l'eau de sureau, les extraits de ginkgo biloba, les extraits d'arnica, les extraits d'origan, les extraits de thé vert, les extraits de fleurs de nénuphar, les extraits d'Iris, les extraits d'écorce de bouleau ;
- l'acide asiatique et les extraits végétaux en contenant comme la Centella Asiatica ;
- le bisabololol ;
- les extraits de fruits, comme l'extrait d'ananas, l'extrait de papaye ;
- les algues notamment du type *Laminaria* (par exemple rouges ou brunes) ;
- les pyrrolidone carboxylates et notamment de zinc (Zn-PCA) ou de cuivre (Cu-PCA);
- les huiles d'origine végétale, comme l'huile de graine de canola et l'huile de karité ;
- les huiles essentielles, par exemple de coriandre, de mélisse, de lavande, de menthe, de camomille et leurs mélanges ;
- l'acide acexamique et l'acide transexamique (acide trans-4, aminométhylcyclohexane carboxylique) ;
- l'acide ursolique et les extraits en contenant comme l'extrait de feuille de romarin ;
- les polysaccharides contenant du fucose, comme le FUCOGEL 1000, vendu par Solabia (solution aqueuse comprenant 1% de matière sèche de polysaccharide comprenant du fucose, du galactose et de l'acide galacturonique) ;
- les électrolytes et en particulier un mélange aqueux comprenant de 30 à 35 % du chlorure de magnésium, de 20 à 28 % de chlorure de potassium, de 3 à 10 % de chlorure de sodium, de 0,2 à 1 % de chlorure de calcium, de 0,1 à 0,6 % de bromure de magnésium et de 0,1 à 0,5 % d'insolubles, le dit mélange étant ici appelé "mélange des sels de la mer Morte" (« Dead Sea Bath Salts ») car il correspond aux principaux sels contenus dans la mer Morte ;
- les galactolipides par exemple issus d'avoine, tels que par exemple le digalactosyl diglycéride ou le monogalactosyl diglycéride ;
- les acides aminés, leurs dérivés et leurs sels, tels que le sel de sodium d'aminoacides greffés sur des chaînes cocoyle, commercialisé sous forme d'un mélange sous la dénomination SEPICALM S par la société SEPPIC, le capryloylglycine commercialisé sous la dénomination LIPACIDE C8G par la société SEPPIC et le mélange de capryloylglycine, de cannelle et de sarcosine commercialisé sous la dénomination SEPICONTROL A5 par la société SEPPIC ;
- les antagonistes de TNF-alpha tels que la lisophylline, l'A802715, la sulfasalazine, le CDP-571 (anticorps anti-TNF-alpha), le MDL-201112 ;
- les antagonistes de substance P tels que le sendide, le spantide II, et les peptides décrits dans le document EP-A-680749, les extraits de bactérie filamenteuse décrits dans le document EP-A-761204 ;
- et leurs mélanges.

La quantité d'agent(s) apaisant(s) peut aller par exemple de 0,001 à 20 % en poids et de préférence de 0,01 à 15 % en poids par rapport au poids total de la composition.

De façon connue, la composition cosmétique ou dermatologique de l'invention peut contenir également des adjuvants habituels dans le domaine cosmétique ou dermatologique, tels que les gélifiants hydrophiles ou lipophiles, les actifs hydrophiles ou lipophiles autres que ceux cités ci-dessus, les conservateurs, les antioxydants, les solvants, les parfums, les charges, les filtres solaires lipophiles ou hydrophiles, les bactéricides, les absorbeurs d'odeur, les matières colorantes, les sels, les polymères (par exemple Acrylates/Dimethicone Copolymer commercialisé sous la dénomination KP-561 par Shin-Etsu, comme dispersant). Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

La composition utilisée selon l'invention peut contenir un ou plusieurs filtres U.V. (ou filtres solaire) qui peuvent être un filtre chimique ou un filtre physique ou un mélange de tels filtres.

On peut citer par exemple comme filtres U.V. l'octylméthoxycinnamate vendu notamment par la société Hoffmann-Laroche sous la dénomination Parsol MCX.

La composition selon l'invention convient, en fonction des agents actifs contenus dans cette composition, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique, dans le maquillage de la peau, des cils et des muqueuses (lèvres). Ainsi, quand elle contient des actifs anti-âge, elle peut être destinée à lutter contre le vieillissement de la peau, et en particulier contre les rides et/ou ridules de la peau, et à donner un teint lisse. Quand elle contient des tensioactifs et notamment des tensioactifs détergents, elle peut être destinée au nettoyage et/ ou au démaquillage de la peau, des cheveux ou de toute autre matière kératinique.

La composition de l'invention peut ainsi constituer une composition de traitement ou de soin de la peau (y compris le cuir chevelu), des fibres kératiniques (cheveux, cils, sourcils), des ongles ou des lèvres, ou une composition de protection solaire ou de bronzage artificiel, ou encore un produit nettoyant ou démaquillant de la peau, des cheveux, des sourcils ou des cils, un produit déodorant ou encore un composé parfumant. Elle est alors généralement non colorée ou faiblement colorée, et elle peut contenir éventuellement des actifs cosmétiques ou dermatologiques. Elle peut alors être utilisée comme base de soin pour la peau ou les lèvres (baumes à lèvres, protégeant les lèvres du froid et/ou du soleil et/ou du vent), comme crème de soin de jour ou de nuit pour la peau du visage et/ou du corps. Elle peut, en outre, se présenter sous forme de shampooing traitant ou non, colorant ou non, et de composé après shampooing.

La composition selon l'invention peut également constituer une composition cosmétique colorée et notamment une composition de maquillage de la peau, des fibres kératiniques (cheveux ou cils) et/ou des muqueuses, en particulier un fond de teint, un blush, un fard à joues ou à paupières, un mascara, un eye-liner, un composé anti-cernes en stick, un vernis à ongle, un rouge à lèvres ou un brillant à lèvres, présentant éventuellement des propriétés de soin ou de traitement, un tatouage corps.

Ainsi, l'invention a encore pour objet l'utilisation cosmétique de la composition telle que définie ci-dessus, pour le soin, le traitement, le nettoyage et/ou le maquillage des matières kératiniques (peau y compris le cuir chevelu, fibres kératiniques et muqueuses).

La présente invention concerne aussi un procédé de traitement cosmétique, caractérisé en ce qu'il met en oeuvre la composition cosmétique selon l'invention.

Préférentiellement, le procédé de traitement cosmétique consiste à appliquer sur la peau, sur le cuir chevelu, et/ou sur les muqueuses, une composition telle que décrite ci-dessus.

Les exemples qui suivent servent à illustrer l'invention sans toutefois présenter un caractère limitatif. Les noms sont, selon le cas, en noms chimiques ou noms CTFA (International Cosmetic Ingredient Dictionary and Handbook) et les quantités en pourcentage en poids sauf mention contraire.

### Exemple 1 : Emulsion H/E

### Phase A1

- Glycérine 7 %
- EDTA (sel de sodium) 0,05 %
- Phénoxyéthanol / parabens (conservateurs) 1 %
- Eau qsp 100 %

### Phase A2

- Acide salicylique 2 %
- Triéthanolamine 2,05 %
- Eau 7 %

### Phase B1

- Alcool cétéarylique 1,2 %
- Oleth-12 0,3 %
- Alcool stéarylique 1 %
- Glyceryl stearate / PEG 100 stéarate (Arlacel 165) 2,5 %
- Polyisobutène hydrogéné 3 %
- Octylméthoxycinnamate (Parsol MCX) 5 %
- Acrylates/Dimethicone Côpolymer (KP-561) (dispersant) 0,6 %

### Phase B2

- Parfum 0,5 %
- Cyclopentasiloxane 7 %
- Octoxyglycérine (Sensiva SC50) 0,5 %

### Phase C

- Sepigel 305 (SEPPIC) 0,7 %

### Phase D

- Fibres de polyamide (Nylon-66) 8 %

### Phase E

- Amidon modifié (DRY FLO) 3 %

Mode opératoire : on mélange les phases A1 et A2 préalablement chauffées à 80°C, et on introduit dans le mélange sous agitation la phase B1 préalablement chauffée à 80°C. On obtient une émulsion fluide que l'on refroidit à 60°C avant d'introduire la phase B2 puis la phase D, et, après refroidissement à 40°C, la phase E, et on agite.

On obtient une crème blanche non irritante et apte à traiter les peaux grasses.

### Exemple 2 : Emulsion H/E

### Phase A

- Glycérine 7 %
- EDTA (sel de sodium) 0,05 %
- Phénoxyéthanol 1 %
- Eau qsp 100 %

### Phase B1

- Alcool cétéarylique 1,2 %
- Oleth-12 0,3 %
- Alcool stéarylique 1 %
- Glyceryl stearate / PEG 100 stéarate (Arlacel 165) 2,5 %
- Polyisobutène hydrogéné 3 %
- Octylméthoxycinnamate (Parsol MCX) 5 %
- Acrylates/Dimethicone Copolymer (KP-561) (dispersant) 0,6 %

### Phase B2

- Parfum 0,5 %
- Cyclopentasiloxane 7 %

### Phase C

- Sepigel 305 (SEPPIC) 0,7 %

### Phase D

- Capryloylglycine 0,5 %
- Eau 2 %

### Phase E

- Fibres de polyamide (Nylon-66) 8 %

Le mode opératoire est identique à celui de l'exemple 1.

On obtient une crème blanche non irritante et apte à traiter les peaux grasses.

**Exemple comparatif:** on a préparé une composition identique à celle de l'exemple 2 mais sans fibres. Il a été réalisé un test sur un panel de 5 personnes, consistant à appliquer en hémi-visage (application simultanée des deux compositions, l'une sur une moitié de visage, l'autre sur la deuxième moitié du visage) la composition de l'exemple 2 et celle de l'exemple comparatif, l'application étant faite sous forme de masque à raison de 10 mg/cm² de composition. Ce test a montré que pour toutes les personnes du panel, la composition de l'exemple 2 était beaucoup plus confortable que celle de l'exemple comparatif (pas ou pratiquement pas de picotements avec la composition selon l'invention et beaucoup de picotements avec la composition de l'exemple comparatif).

## Revendications

1. Utilisation cosmétique de fibres comme agent anti-irritant dans une composition cosmétique.

2. Utilisation de fibres comme agent anti-irritant pour la préparation d'une composition pour application topique destinée à traiter les matières kératiniques.

3. Utilisation cosmétique d'une quantité efficace de fibres pour diminuer l'effet irritant d'une composition cosmétique contenant un ou plusieurs composés à effet secondaire irritant.

4. Utilisation d'une quantité efficace de fibres pour la préparation d'une composition dermatologique contenant un ou plusieurs composés à effet secondaire irritant, les fibres étant destinées à diminuer cet effet irritant.

5. Utilisation selon l'une quelconque des revendications précédentes, **caractérisée en ce que** les fibres ont une longueur (L) allant de 1 µm à 10 mm.

6. Utilisation selon l'une quelconque des revendications précédentes, **caractérisée en ce que** les fibres ont une section comprise dans un cercle de diamètre (D) allant de 1 nm à 100 µm.

7. Utilisation selon l'une quelconque des revendications précédentes, **caractérisée en ce que** les fibres ont un facteur de forme (L/D) allant de 5 à 150.

8. Utilisation selon l'une quelconque des revendications précédentes, **caractérisée en ce que** les fibres ont un titre allant de 0,15 à 30 deniers.

9. Utilisation selon l'une quelconque des revendications précédentes, **caractérisée en ce que** les fibres sont choisies parmi les fibres de soie, de coton, de laine, de lin, de cellulose extraites notamment du bois, des légumes ou des algues, de polyamide (Nylon®), de cellulose modifiée, de poly-p-phénylène téréphtalamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges.

10. Utilisation selon l'une quelconque des revendications précédentes, **caractérisée en ce que** les fibres sont enrobées et/ou fonctionnalisées.

11. Utilisation selon l'une quelconque des revendications précédentes, **caractérisée en ce que** les fibres sont choisies parmi les fibres de polyamide, les fibres de poly-p-phénylène téréphtalamide, les fibres de coton et leurs mélanges.

12. Utilisation selon l'une quelconque des revendications précédentes, **caractérisée en ce que** les fibres sont présentes en une quantité allant de 0,01 à 50 % en poids et de préférence de 1 à 20 % en poids par rapport au poids total de la composition.

13. Utilisation selon l'une quelconque des revendications 3 à 12, **caractérisée en ce que** le composé à effet secondaire irritant est choisi parmi les actifs, les conservateurs, les tensioactifs, les parfums, les solvants, les propulseurs, et leurs mélanges.

14. Utilisation selon l'une quelconque des revendications 3 à 13, **caractérisé en ce que** le composé à effet secondaire irritant est choisi parmi les α-hydroxy-acides, les β-hydroxy-acides, les α-céto-acides, les β-céto-acides, les rétinoïdes, les anthralines, les anthranoïdes, les peroxydes, le minoxidil et ses dérivés, les sels de lithium, les teintures ou colorants capillaires, les solutions alcooliques parfumantes, les agents antitranspirants, les actifs dépilatoires ou de permanentes, les dépigmentants, la capsaïcine, les actifs antipoux, les détergents, les bactéricides, les tensioactifs anioniques, et leurs mélanges.

15. Utilisation selon l'une quelconque des revendications 3 à 14, **caractérisée en ce que** le composé à effet secondaire irritant est choisi parmi les acides glycolique, lactique, malique, citrique, tartrique, mandélique, et leurs dérivés, l'acide salicylique et ses dérivés, l'acide n-octanoyl-5-salicylique et l'acide n-dodécanoyl-5-salicylique et leurs esters, le rétinol et ses esters, le rétinal, l'acide rétinoïque et ses dérivés, le phénoxyéthanol, l'octoxyglycérine, le caprylyl-glycol, les alkyl sulfates et alkyl éther sulfates et leurs sels, et leurs mélanges.

16. Utilisation selon l'une quelconque des revendications 3 à 15, **caractérisée en ce que** la quantité de composé(s) à effet secondaire irritant va de 0,0001 à 50 % en poids et de préférence de 0,01 à 30 % en poids par rapport au poids total de la composition.

17. Utilisation selon l'une quelconque des revendications précédentes, **caractérisée en ce que** la composition contient en outre un milieu physiologiquement acceptable.

18. Composition pour application topique, **caractérisée en ce qu'**elle comprend des fibres et un ou plusieurs composés à effet secondaire irritant choisi parmi les α-hydroxy-acides, les β-hydroxy-acides, les α-céto-acides, les β-céto-acides, les anthralines, les anthranoïdes, les peroxydes, le minoxidil et ses dérivés, les sels de lithium, les agents antitranspirants, les actifs dépilatoires ou de permanentes, les dépigmentants, la capsaïcine, les actifs antipoux, les tensioactifs anioniques, et leurs mélanges.

19. Composition selon la revendication précédente, **caractérisée en ce que** le composé à effet secondaire irritant est choisi parmi les acides glycolique, lactique, malique, citrique, tartrique, mandélique et leurs dérivés, l'acide salicylique et ses dérivés, l'acide ascorbique et ses dérivés, les alkyl sulfates et alkyl éther sulfates et leurs sels, et leurs mélanges.

20. Composition selon la revendication précédente, **caractérisée en ce que** les dérivés de l'acide salicylique sont choisis parmi l'acide n-octanoyl-5-salicylique et l'acide n-dodécanoyl-5-salicylique.

21. Composition selon l'une quelconque des revendications 18 à 20, **caractérisée en ce qu'**elle contient au moins deux composés à effet secondaire irritant.

22. Composition selon l'une quelconque des revendications 18 à 21, **caractérisée en ce que** la quantité de composé à effet secondaire irritant va de 0,0001 à 50 % en poids et de préférence de 0,01 à 30 % en poids par rapport au poids total de la composition.

23. Composition pour application topique, contenant dans un milieu physiologiquement acceptable, des fibres et au moins un agent apaisant choisi parmi I'allantoïne ; l'acide bêta-glycyrrhétinique et les extraits et complexes en contenant ; les planctons ; l'escine et les extraits végétaux en contenant ; les dérivés de xanthine ; les eaux et extraits de fleurs et de plantes ; l'acide asiatique et les extraits végétaux en contenant ; le bisabololol ; les extraits de fruits ; les algues ; les pyrrolidone carboxylates ; les huiles d'origine végétale ; les huiles essentielles ; l'acide acexamique et l'acide transexamique ; l'acide ursolique et les extraits en contenant ; les polysaccharides contenant du fucose ; les électrolytes ; les galactolipides ; les acides aminés, leurs dérivés et leurs sels ; les antagonistes de TNF-alpha ; les antagonistes de substance P ; et leurs mélanges.

24. Composition selon la revendication précédente, **caractérisée en ce que** la quantité d'agent(s) apaisant(s) va de 0,001 à 20 % en poids et de préférence de 0,01 à 15 % en poids par rapport au poids total de la composition.

25. Composition selon la revendication 23 ou 24, **caractérisée en ce qu'**elle contient en outre au moins un composé à effet secondaire irritant.

26. Composition selon la revendication précédente, **caractérisée en ce que** le composé à effet secondaire irritant est choisi parmi les α-hydroxy-acides, les β-hydroxy-acides, les α-céto-acides, les β-céto-acides, les rétinoïdes, les anthralines, les anthranoïdes, les peroxydes, le minoxidil et ses dérivés, les sels de lithium, les teintures ou colorants capillaires, les solutions alcooliques parfumantes, les agents antitranspirants, les actifs dépilatoires ou de permanentes, les dépigmentants, la capsaïcine, les actifs antipoux, les détergents, les bactéricides, les tensioactifs anioniques, et leurs mélanges.

27. Composition selon l'une quelconque des revendications 18 à 26, **caractérisée en ce que** les fibres sont conformes aux fibres selon l'une quelconque des revendications 5 à 11.

28. Composition selon l'une quelconque des revendications 18 à 27, **caractérisée en ce que** les fibres sont présentes en une quantité allant de 0,01 à 50 % en poids et de préférence de 1 à 20 % en poids par rapport au poids total de la composition.

29. Composition selon l'une quelconque des revendications 18 à 28, **caractérisée en ce que** la composition est sous forme d'une émulsion.

30. Composition selon l'une quelconque des revendications 18 à 29, **caractérisée en ce qu'**elle constitue une composition de soin, de traitement et/ou de maquillage de la peau, des fibres kératiniques et/ou des muqueuses.

31. Procédé de traitement cosmétique, **caractérisé en ce qu'**il met en oeuvre la composition cosmétique selon l'une des revendications 18 à 29.

32. Utilisation cosmétique de la composition cosmétique selon l'une des revendications 18 à 29, pour le soin, le traitement, le nettoyage et/ou le maquillage des matières kératiniques.

33. Procédé cosmétique pour diminuer ou supprimer l'effet irritant cutané d'au moins un composé à effet secondaire irritant présent dans une composition cosmétique, **caractérisé en ce que** l'on utilise une quantité efficace de fibres.
